(19) Europäisches Patentamt
European Patent Office
Office européen des brevets

(11) Publication number: **0 211 502 B1**

(12) **EUROPEAN PATENT SPECIFICATION**

(45) Date of publication of patent specification: **25.09.91**

(51) Int. Cl.$^5$: **A61K 31/20**, A61K 31/16, A61K 31/23

(21) Application number: **86304860.9**

(22) Date of filing: **24.06.86**

(54) **Use of metabolites of linoleic or linolenic acid in the treatment of premenstrual syndrome.**

(30) Priority: **04.07.85 GB 8516906**

(43) Date of publication of application:
**25.02.87 Bulletin 87/09**

(45) Publication of the grant of the patent:
**25.09.91 Bulletin 91/39**

(84) Designated Contracting States:
**AT BE CH DE FR GB IT LI LU NL SE**

(56) References cited:
**EP-A- 0 003 407**
**EP-A- 0 181 689**

**R.BERKOW et al.: "THE MERCK MANUAL OF DIAGNOSIS AND THERAPY", 1987, 15th edition, pages 1710-1711, Merck & Co., Inc., Rahway, N.J., US**

(73) Proprietor: **EFAMOL HOLDINGS PLC**
**Efamol House Woodbridge Meadows**
**Guildford Surrey GU1 1BA(GB)**

(72) Inventor: **Horrobin, David Frederick**
**c/o Efamol Limited Efamol House Woodbridge Meadows**
**Guildford Surrey, GU1 1BA(GB)**

(74) Representative: **Miller, Joseph et al**
**J. MILLER & CO. Lincoln House 296-302 High Holborn**
**London WC1V 7JH(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

## Description

The essential fatty acids (EFAs) are of two types, the n-3 (or omega-3) series derived from alpha-linolenic acid and the n-6 (or omega-6) series derived from linoleic acid. Linoleic acid and alpha-linolenic acid are like vitamins in that they cannot be manufactured in the body and therefore must be provided in the diet. The body can metabolise them along the pathways below and such metabolism is believed to be essential if they are to fulfil their functions. The pathways, sharing it is believed common enzymes, are:

| n-6 | n-3 |
|---|---|
| 18:2 delta-9,12(linoleic acid) | 18:3 delta-9,12,15 (alpha-linolenic acid) |
| ↓ delta-6 desaturase | ↓ |
| 18:3 delta-6,9,12 (gamma-linolenic acid) | 18:4 delta-6,9,12,15 |
| ↓ elongation | ↓ |
| 20:3 delta-8,11,14 (dihomo-gamma-linolenic acid) | 20:4 delta-8,11,14,17 |
| ↓ delta-5 desaturase | ↓ |
| 20:4 delta-5,8,11,14 (arachidonic acid) | 20:5 delta-5,8,11,14,17 |
| ↓ elongation | ↓ |
| 22:4 delta-7,10,13,16 (adrenic acid) | 22:5 delta-7,10,13,16,19 |
| ↓ delta-4 desaturase | ↓ |
| 22:5 delta-4,7,10,13,16 | 22:6 delta-4,7,10,13,16,19 |

The pathways are not normally reversible nor, in man, are n-3 and n-6 series acids interconvertible.

The acids, which naturally are of the all-cis configuration, are systematically named as derivatives of the corresponding octadecanoic, eicosanoic or docosanoic acids e.g. delta-9,12-octadecadienoic acid or delta-4,7,10,13,16,19-docosahexaenoic acid, but numerical designation such as, correspondingly, 18:2 n-6 or 22:6 n-3 is convenient. Initials for example, DHA for 22:6 n-3 (docosahexaenoic acid), are also used but do not served when n-3 and n-6 acids of the same chain length and degree of unsaturation exist. Trivial names in more or less common use in the n-6 series are as shown. Of the n-3 series only 18:3 n-3 has a commonly used trivial name, alpha-linolenic acid. It was characterised earlier than gamma-linolenic acid and reference in the literature simply to linolenic acid, especially in the earlier literature is to the alpha-acid.

In the body, the n-3 acids are metabolised preferentially and as a result, in plasma for example, levels of alpha-linolenic acid (18:3 n-3) are low and 18:4 n-3 and 20:4 n-3 are in trace amounts only. In contrast the n-6 acids are normally present in moderate amounts, though gamma-linolenic acid (GLA) is at low levels being apparently converted to dihomo-gamma-linolenic acid (DGLA) more rapidly than its relatively slow production from linoleic acid. In both series the elongation stages in the metabolic pathways are much more rapid than the desaturations.

Particular significance of the n-6 series acids lies in prostaglandin (PG) synthesis, the outline of which is believed to be as shown in the following diagram:

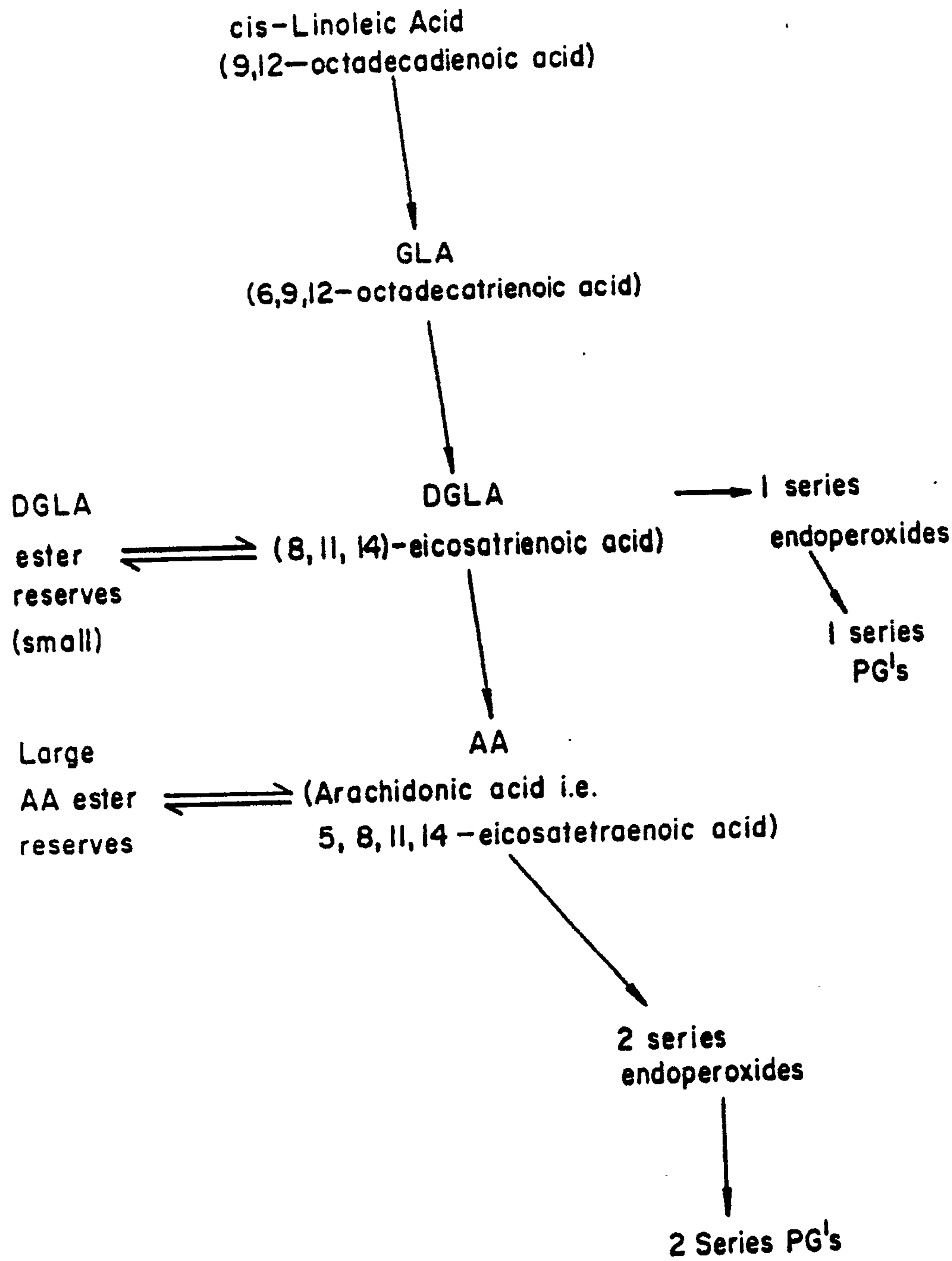


The broad outline of this pathway is well known, and it brings out clearly that a major function of essential fatty acids is to act as precursors for 1-series PGs formed from DGLA and 2-series PGs formed from arachidonic acid (AA). Further it has recently been found that the 22:4 n-6 acid produced from arachidonic acid gives rise to a series of homo-2-series PGs, though their importance is as yet unknown.

DGLA is the key substance. GLA is almost completely and very rapidly converted in the body to DGLA and so for practical purposes the oral administration of DGLA and GLA amounts to the same thing. DGLA can be converted to a storage form or to PGs of the 1-series or, through arachidonic acid, to PGs of the 2-series.

Considering dietary requirements, it is well known, for example, that linoleic acid cannot be made by the body and so must be taken in the diet. However, it has been generally thought that the body can metabolise linoleic acid to all the other n-6 acids and therefore that provided linoleic acid intake is adequate, no lack of the other n-6 acids will be found.

In previous patent applications (for example Published European Patent Application No A 0 003 407,

U.S. Patent No. 4 273 763; Published European Patent Application No. A 0 004 770, U.S. Patent No. 4 309 415; Published Europran Patent Application No. 0 019 423, U.S. Patent No. 4 388 324) it has, however, been pointed out that the first enzyme in the pathway, the delta-6 desaturase, which, for example, converts linoleic acid to GLA is not fully effective in a variety of conditions. The administration of GLA or DGLA acid or both has been suggested and has been successful in treating a variety of clinical conditions.

In the above patent applications attention is primarily paid to the function of essential fatty acids in prostaglandin metabolism and in particualr to their role in securing a proper balance between 1-series and 2-series PGs.

The applicants are, however, becoming increasingly aware of the significance of the essential fatty acids in themselves as set out above. Considerable general interest has been shown in them in recent years, primarily in the acids of the n-6 series both as such and in relation to prostaglandin metabolism, but also in the acids of the n-3 series. The n-6 acids in particular are required in the body for the structure of membranes in and around cells, being believed to be necessary for maintaining normal flexibility, fluidity and permeability of such membranes.

Generally as regards PGs, as appears from the earlier patent applications referred to, and from other publications by the inventor, the actions of the 1-series PGs and other metabolic products derived from DGLA are almost all either desirable or neutral, but the actions of the 2-series PGs and other metabolic products derived from arachidonic acid are very mixed, some being desirable and some being highly undesirable. Studies of the interactions between the metabolism of the n-6 acids and that of the n-3 acids have shown that elongation reactions (e.g. GLA to DGLA) are highly efficient and there is very little competition either way. In contrast, the two series of fatty acids are in competition in the desaturation processes. The n-3 fatty acids interfere with both delta-6 and delta-5 desaturation in the n-6 series. This competition seems to occur even when the n-3 fatty acid is not actually a substrate for the enzyme concerned. For example, 20:5 n-3 competitively inhibits the delta-6 desaturation forming GLA from linoleic acid and overall the presence of n-3 fatty acids in a combination leads to some inhibition of the conversion of DGLA to arachidonic acid by the delta-5 desaturase. Thus as a result of the presence of n-3 EFAs, the efficiency of either GLA and DGLA in increasing the ratio of DGLA products (1-series PGs) to arachidonic acid products (2-series PGs) will be increased, quite apart from the value of the n-3 acids in themselves.

In studies of the very common condition in women usually referred to as premenstrual syndrome, we have found that there is a group unresponsive to GLA or DGLA acid alone. Many of these women can, however, be helped by giving these acids in combination with n-3 series acids. The malfunction leading to the syndrome in the women concerned is not yet clear, and any combination of n-3 and n-6 essential fatty acids is believed to be of value. However, it is believed that the delta-6 desaturase converting linoleic acid to GLA and alpha-linolenic acid to 18:4 n-3 is deficient, giving inadequate conversion, and that accordingly the combination should preferably comprise GLA or a higher n-6 acid and 18:4 n-3 or a higher n-3 acid.

## DETAILED STATEMENT OF THE INVENTION

In the light of the discussion above the present invention may be summarised as:-

(i) The use for the manufacture of a medicament for the treatment of amenable pre-menstrual syndrome of one or more of linoleic acid and the metabolites of linoleic acid (GLA, DGLA, AA, 22:4 n-6 or 22:5 n-6) together with one or more of alpha-linolenic acid and the metabolites of alpha-linolenic acid (18:4 n-3, 20:4 n-3, 20:5 n-3, 22:5 n-3 or 22:6 n-3), as such or in the form of an ester, salt, amide or other derivative convertible in the body thereto, alone or in an acceptable pharmaceutical carrier or diluent.

The acids may be administered in the form of the acid itself or as an ester, amide, salt or any other functional derivative capable of being converted to a biologically active form of the acid within the body and may be from natural or synthetic sources.

Judging by their presence in various body tissues, the important n-3 EFAs are 20:5 n-3 and 22:6 n-3. One therefore wants most preferably to use combinations of either 18:4 n-3 or 20:4 n-3 which can give rise to 20:5 n-3 and 22:6 n-3, or of those acids themselves, with either GLA or DGLA.

Doses for each acid are from 1 mg to 50 g per day, preferably 50 gm to 5 g per day, conveniently in conventional gelatine capsules.

## FORMS AND SOURCE OF GAMMA-LINOLENIC AND OTHER ACIDS

Conventional sources of the acids include Evening Primrose (Oenothera) oil containing about 9% of GLA and oils from marine or migratory fish which contain substantial amounts of 20:5n-3 and 22:6n-3.

Suitable physiologically functional derivatives, convertible in the body to the acids to enter the

metabolic pathways given earlier herein, are physiologically acceptable salts, esters (particularly glycerides and simple $C_1$ $C_4$ alkyl esters), amides and phospholipids. Indirect identification of useful derivatives is by their having the valuable effect in the body of the acid itself, but conversion can be shown directly by gas chromatographic analysis of concentrations in blood, body fat, or other tissue by standard techniques for example those of Pelick et al. p. 23, "Analysis of Lipids and Lipoproteins" Ed. Perkins, American Oil Chemists Society, Champaign, Illinois, U.S.A.

DIETARY COMPOSITIONS

The invention is chiefly described in terms of pharmaceutical compositions, but it will be understood that GLA and the other acids being in the nature of dietary supplements, could be incorporated in a dietary margarine or other foodstuffs: such foodstuffs, possibly containing other active materials and generally referred to in this description as dietary or pharmaceutical compositions, are within the purview of the invention and thus of the term pharmaceutical compositions used in the claims.

VETERINARY APPLICATIONS

It will be understood that where a disorder of a kind calling for treatment in animals arises, the invention while described primarily in terms of human medicine is equally applicable in the veterinary field.

PHARMACEUTICAL PRESENTATION

The compositions are conveniently in a form suitable for oral, rectal, parenteral or topical administration in a suitable pharmaceutical vehicle, as discussed in detail for example in U.K. Patent Specification No. 1 082 624 and in any case very well known generally for any particular kind of preparation. Thus for example tablets, capsules, ingestible liquid or powder preparations, creams and lotions for topical application, or suppositories, can be prepared as required. Injectable solutions of hydrolysed Oenothera oil may be prepared using albumin to solubilise the free acid.

Advantageously a preservative is incorporated into the preparations. Alpha-tocopherol in a concentration of about 0.1% by weight has been found suitable for the purpose.

It will be understood that the absolute quantity of active ingredients present in any dosage unit should not exceed that appropriate to the rate and manner of administration to be employed but on the other hand should also desirably be adequate to allow the desired rate of administration to be achieved by a small number of doses. The rate of administration will moreover depend on the precise pharmacological action desired.

The following are specific examples of the invention, against amenable premenstrual syndrome:

EXAMPLE 1

A capsule containing 60% Evening Primrose oil and 40% mackerel oil administered in 0.5g capsules six times per day.

EXAMPLE 2

A capsule containing 70% Evening Primrose oil and 30% salmon oil administered in 0.5g capsules eight times per day.

EXAMPLE 3

A 0.25g capsule containing 150mg of GLA and 100mg of 20:5n-3 administered 3 times per day.

EXAMPLE 4

A capsule containing 50mg of DGLA, 50mg of AA, 20mg of 22:4n-6, 20mg of 22:5n-6, 50mg of20:5n-3, 20mg of 22:5n-3 and 20mg of 22:6n-3 taken four times a day.

**Claims**

**Claims for the following Contracting States : BE, FR, DE, IT, LU, NL, CH/LI, GB, SE**

1. The use for the manufacture of a medicament for the treatment of amenable pre-menstrual syndrome of one or more of linoleic acid and the metabolites of linoleic acid (GLA, DGLA, AA, 22:4 n-6 or 22:5 n-6) together with one or more of alpha-linolenic acid and the metabolites of alpha-linolenic acid (18:4 n-3, 20:4 n-3, 20:5 n-3, 22:5 n-3 or 22:6 n-3), as such or in the form of an ester, salt, amide or other derivative convertible in the body thereto, alone or in an acceptable pharmaceutical carrier or diluent.

2. The use set out in claim 1, wherein the metabolites of linoleic acid used are one or both of GLA and DGLA.

3. The use as set out in claim 1 or 2, wherein the amounts of each said acid are 1 mg to 50 g, preferably 50 mg to 5 g per unit dose.

**Claims for the following Contracting State : AT**

1. A method for producing a pharmaceutical composition for the treatment of amenable pre-menstrual syndrome characterised in that one or more of linoleic acid and the metabolites of linoleic acid (GLA, DGLA, AA, 22:4 n-6 or 22:5 n-6) together with one or more of alpha-linolenic acid and the metabolites of alphalinolenic acid (18:4 n-3, 20:4 n-3, 20:5 n-3, 22:5 n-3 or 22:6 n-3), as such or in the form of an ester, salt, amide or other derivative convertible in the body thereto, are formulated in a dosage unit form alone or in an acceptable pharmaceutical carrier or diluent.

2. The method set out in claim 1, wherein the metabolites of linoleic acid used are one or both of GLA and DGLA.

3. The method as set out in claim 1 or 2, wherein the amounts of each said acid are 1 mg to 50 g, preferably 50 mg to 5 g per unit dose.

**Revendications**

**Revendications pour les Etats contractants suivants : BE, FR, DE, IT, LU, NL, CH/LI, GB, SE**

1. Emploi pour la préparation d'un médicament destiné au traitement du syndrome prémenstruel justiciable d'un traitement, d'une ou plusieurs des substances acide linoléique et métabolites de l'acide linoléique (GLA, DGLA, AA, 22:4 n-6 ou 22:5 n-6) associées à une ou plusieurs des substances acide alpha-linolénique et métabolites de l'acide alpha-linolénique (18:4 n-3, 20:4 n-3, 20:5 n-3, 22:5 n-3 ou 22:6 n-3), en tant que telles ou sous la forme d'un ester, d'un sel, d'un ami de ou d'un autre dérivé convertibles en ces substances dans l'organisme, seules ou dans un excipient pharmaceutique acceptable ou un diluant.

2. Emploi selon la revendication 1, dans lequel les métabolites de l'acide linoléique utilisés sont soit GLA, soit DGLA ou l'un et l'autre.

3. Emploi selon la revendication 1 ou 2, dans lequel les quantités de chacun desdits acides sont comprises entre 1 mg et 50 g, de préférence entre 50 mg et 5 g par dose unitaire.

**Revendications pour l'Etat contractant suivant : AT**

1. Procédé de production d'une composition pharmaceutique destinée au traitement du syndrome prémenstruel justiciable d'un traitement, caractérisé en ce qu'une ou plusieurs des substances acide linoléique et métabolites de l'acide linoléique (GLA, DGLA, AA, 22:4 n-6 ou 22:5 n-6) associées à une ou plusieurs des substances acide alpha-linolénique et métabolites de l'acide alpha-linolénique (18:4 n-3, 20:4 n-3, 20:5 n-3, 22:5 n-3 ou 22:6 n-3), en tant que telles ou sous la forme d'un ester, d'un sel, d'un amide ou d'un autre dérivé convertibles dans l'organisme en ces substances, sont formulées sous la forme d'unités posologiques, seules ou dans un excipient pharmaceutique acceptable ou un diluant.

2. Procédé selon la revendication 1, dans lequel les métabolites de l'acide linoléique utilisés sont GLA ou DGLA ou l'un et l'autre.

3. Procédé selon la revendication 1 ou 2, dans lequel les quantités de chacun desdits acides sont

comprises entre 1 mg et 50 g, de préference entre 50 mg et 5 g par dose unitaire.

**Patentansprüche**

**Patentansprüche für folgende Vertragsstaaten : BE, FR, DE, IT, LU, NL, CH/LI, GB, SE**

1. Die Verwendung zur Herstellung eines Medikamentes für die Behandlung eines zugänglichen prämenstruellen Syndroms von einer oder mehr Linolensäure und der Metaboliten von Linolensäure (GLA, DGLA, AA, 22:4 n-6 oder 22:5 n-6) zusammen mit einer oder mehr Alpha-Linolensäure und den Metaboliten von Alpha-Linolensäure (18:4 n-3, 20:4 n-3, 20:5 n-3, 22:5 n-3 oder 22:6 n-3), als solche oder in Form eines Esters, Salzes, Amides oder anderen Derivates, das sich im Körper dazu umwandelt, allein oder in einer akzeptablen pharmazeutischen Trägersubstanz oder einem Verdünnungsmittel.

2. Die Verwendung gemäß Anspruch 1, worin ein oder beide der verwendeten Metabolite von Linolensäure aus GLA und DGLA sind.

3. Die Verwendung gemäß Anspruch 1 oder 2, worin die Mengen jeder besagten Säure 1 mg bis 50 g betragen, vorzugsweise 50 mg bis 5 g pro Einheitsdosis.

**Patentansprüche für folgenden Vertragsstaat : AT**

1. Verfahren zur Herstellung einer pharmazeutischen Zusammensetzung zur Behandlung eines zugänglichen prämenstruellen Syndroms, dadurch gekennzeichnet, daß eine oder mehr Linolensäure und die Metaboliten der Linolensäure (GLA, DGLA, AA, 22:4 n-6 oder 22:5 n-6) zusammen mit einer oder mehr Alpha-Linolensäure und den Metaboliten von Alpha-Linolensäure (18:4 n-3, 20:4 n-3, 20:5 n-3, 22:5 n-3 oder 22:6 n-3) , als solche oder in Form eines Esters, Salzes, Amides oder anderen Derivates, das sich im Körper dazu umwandelt, in Form einer Dosierungseinheit allein oder in einer akzeptablen pharmazeutischen Trägersubstanz oder einem Verdünnungsmittel formuliert werden.

2. Verfahren gemäß Anspruch 1, worin eine oder beide der verwendeten Metabolite der Linolensäure aus GLA und DGLA sind.

3. Verfahren gemäß Anspruch 1 oder 2, worin die Mengen jeder besagten Säure 1 mg bis 50 g betragen, in bevorzugter Weise 50 mg bis 5 g pro Einheitsdosis.